# EUROPEAN PATENT APPLICATION

(11) **EP 2 048 156 A1**
(43) Date of publication of application: **15.04.2009**
(21) Application number: 07020025.8
(22) Date of filing: 12.10.2007
(51) Int. Cl.: C07K 14/33, A61K 38/16, A61K 47/48

(54) **Process for providing a temperature-stable muscle relaxant on the basis of the neurotoxic component of botulinum toxin**

(71) Applicant: Merz Pharma GmbH & Co. KGaA, 60318 Frankfurt (DE)
(72) Inventor: Eisele, Karl-Heinz, 70794 Filderstadt (DE); Marx, Matthias, 68199 Mannheim (DE); Taylor, Harold V., 60594 Frankfurt/Main (DE)
(74) Representative: Ricker, Mathias

(57) **Abstract**

The present invention provides a process for providing a muscle relaxant, wherein said muscle relaxant is a reconstituted solution comprising the neurotoxic component of botulinum toxin free of complexing proteins, which exhibits at least one of the following characteristics, more preferably all characteristics a) to d):
a) Stable at storage temperatures above 20°C
b) Stable in the presence of preservatives and/or analgesics
c) Resistant against "freeze and thaw" - cycles
d) Stable if stored in containers of different material.

## Description

### FIELD OF THE INVENTION

The present invention provides a process for providing a muscle relaxant, wherein said muscle relaxant is a reconstituted solution comprising the neurotoxic component of botulinum toxin free of complexing proteins, which exhibits at least one of the following characteristics, more preferably all characteristics a) to d):
a) Stable at storage temperatures above +20°C
b) Stable in the presence of preservatives and/or analgesics
c) Resistant against "freeze and thaw" - cycles
d) Stable if stored in containers of different materials

### BACKGROUND OF THE INVENTION

Botulinum toxin is produced by the bacterium *Clostridium.* There are seven antigenically distinct serotypes of Botulinum toxin, namely Botulinum toxin A, B, C, D, E, F and G. Botulinum toxins are released from lysed Clostridium cultures generally in the form of a complex, i.e. the sub-unit responsible for the toxic properties of the Botulinum toxin (the so-called "neurotoxic component"), is associated with other bacterial proteins, which together form a toxin complex. The molecular weight of this complex may vary from about 300,000 to about 900,000 Da. The complexing proteins are, for example, various hemagglutinins. The proteins of this toxin complex are not toxic themselves but are believed to provide stability to the neurotoxic component and are responsible for oral toxicity in Botulinum intoxications Unlike the toxin complex, the neurotoxic component in its isolated and pure form, i.e. devoid of any completing Clostridium proteins, is acid labile and does not resist the aggressive environment in the gastrointestinal tract.

The neurotoxic component of the Botulinum toxin complex is initially formed as a single polypeptide chain, having in the case of serotype A a molecular weight of approximately 150 kDa. In other serotypes the neurotoxic component has been observed to vary between about 145 and about 170 kDa, depending on the bacterial source. In the case of serotype A, for example, proteolytic processing of the polypeptide results in an activated polypeptide in the form of a dichain polypeptide consisting of a heavy chain and a light chain, which are linked by a disulfide bond. In humans, the heavy chain mediates binding to pre-synaptic cholinergic nerve terminals and internalization of the toxin into the cell. The light chain is believed to be responsible for the toxic effects, acting as zink-endopeptidase and cleaving specific proteins responsible for membrane fusion (SNARE complex) (see e.g. Montecucco C., Shiavo G., Rosetto O: The mechanism of action of tetanus and Botulinum neurotoxins. Arch Toxicol. 1996; 18 (Suppl.): 342-354)).

The neurotoxic subunit of the Botulinum toxin complex is referred herein as the "neurotoxic component" or the "neurotoxic component free of complexing proteins".

The terms "Botulinum toxin" or "Botulinum toxins" as used throughout the present application, refer to the neurotoxic component devoid of any other clostridial proteins, but also to the "Botulinum toxin complex": The term "Botulinum toxin" is used herein in cases when no discrimination between the toxin complex and the neurotoxic component is necessary or desired. The complex usually contains additional, so-called "non-toxic" proteins, which we will refer to as "complexing proteins" or "bacterial proteins".

Despite its toxic effects, Botulinum toxin complex has been used as a therapeutic agent in a large number of diseases. Botulinum toxin serotype A was approved for human use in the United States in 1989 for the treatment of strabism, blepharospasm, and other disorders. It is commercially available as Botulinum toxin A protein complex, for example, under the tradename BOTOX (Allergan Inc.) or under the tradename DYSPORT (Ipsen Ltd). For therapeutic application the complex is injected directly into the muscle to be treated. At physiological pH, the neurotoxic component is released from the protein complex and the desired pharmacological effect takes place A pharmaceutical composition comprising the neurotoxic component of Botulinum toxin type A in isolated form is commercially available in Germany from Merz Pharmaceuticals GmbH under the trademark Xeomin^{®}. The production of the neurotoxic component of Botulinum toxin type A and B are described, for example, in the international patent application WO 00/74703.

With regard to the composition and dosing of the medicament on the basis of Botulinum toxin, and in regard to the composition, dosing and frequency of administration of the medicament on the basis of the neurotoxic component of Botulinum toxin, reference is made to PCT/EP2007/005754.

In addition to the above-recited function of the complexing proteins it has been speculated that they also protect the neurotoxic component of the Botulinum toxin complex from harsh environmental conditions, and that the neurotoxic component as such is highly susceptible to degradation or inactivation or both, especially when subjected to short-term temperature stress, such as storage or transport or both in warm to hot climate or during summer in general, i.e. a temperature above 30°C.

For said reason, utmost care is generally taken in the past to prevent the medicaments on the basis of the Botulinum toxins and those on the basis of the neurotoxic component of Botulinum toxin in particular, from reaching a temperature of above +4°C, e.g. close to +20°C. In most cases, the vials containing the solid dry lyophilisate comprising the Botulinum toxins or the reconstituted solutions thereof were stored frozen around -20°C only (lyophilisate), on ice or at least in a refrigerator (around +4°C). The necessary cooling results in additional costs to those of providing the medicaments.

Furthermore, it was believed prior to the present invention that the reconstituted solution comprising the neurotoxic component of the Botulinum toxin is even more unstable with regard to different storage or transport conditions. Additionally it was thought, that freezing and thawing of the reconstituted solution would lead to a rapid degradation and inactivation of the protein. Therefore, the physician was advised to reconstitute the protein-lyophilisate only just before administering the drug and/or to strictly store it at low temperature as outlined above.

In view of the above situation, the inventors performed studies regarding the stability of muscle relaxants on the basis of Botulinum toxin in the form of a reconstituted solution under different environmental conditions:
a) Storage at temperatures above +20 °C
b) Addition of preservatives and/or analgesics
c) Freeze and thaw - cycles
d) Storage in containers made of different materials

It was surprisingly found that the reconstituted solution comprising the neurotoxic component of Botulinum toxin free of complexing proteins is significantly more stable under these conditions than expected in the art. The hereinunder described invention is based on this finding.

### SUMMARY OF THE INVENTION

The present invention provides a process for providing a muscle relaxant, wherein said muscle relaxant is a reconstituted solution comprising the neurotoxic component of botulinum toxin free of complexing proteins, which exhibits at least one of the following characteristics, more preferably all characteristics a) to d):
a) Stable at storage temperatures above +20°C
b) Stable in the presence of preservatives and/or analgesics
c) Resistant against "freeze and thaw" - cycles
d) Stable if stored in containers of different material

### BRIEF DESCRIPTION OF THE DRAWINGS

Figure 1: Effect of storage with and without preserved saline solution upon the activity of reconstituted Xeomin^{®} and Botox^{®} at +4°C. Storage in polyethylene vessels.
Figure 2: Effect of storage with and without preserved saline solution upon the activity of reconstituted Xeomin^{®} and Botox^{®} at +4°C. Storage in polyethylene syringes with rubber stoppers.
Figure 3: Effect of repeated freezing and thawing upon the activity of reconstituted Xeomin^{®}.

### DETAILED DESCRIPTION OF THE INVENTION

The present invention relates to a process for providing a muscle relaxant at temperatures above +4°C, preferably above +6°C, more preferably above +20°C, wherein said muscle relaxant is a reconstituted solution comprising the neurotoxic component of Botulinum toxin free of complexing agents. Within this invention, the term "providing" includes any kind of provision of the muscle relaxant defined herein, in particular storage, transport, and/or a step within the preparation of said muscle relaxant. The term "providing" also includes steps wherein the muscle relaxant is subjected to a rise in temperature from the frozen (e.g. - 20°C) state to a temperature of above +4°C, preferably above +6°C, more preferably above +20°C. Within this invention, all forms of the neurotoxin component of Botulinum toxin, in particular the various serotypes are to be used, including serotypes A, B, C, D, E, F and G. In a preferred embodiment of the present invention, said neurotoxic component of Botulinum toxin of serotype B is provided at a temperature of above 8°C.In addition thereto, modified as well as recombinantly produced neurotoxic components of Botulinum toxins including the respective mutations, deletions, etc. are also within the scope of the present invention. With respect to suitable mutants, reference is made to WO 2006/027207 A1 and WO 2006/114308 A1, which are fully incorporated by reference herein. Furthermore, within the present invention, mixtures of various serotypes (in the form the neurotoxic component or recombinant form or both forms thereof, e.g. mixtures of Botulinum neurotoxins of types A and B) may be used. The present invention, however, also refers to neurotoxins which are chemically modified, e.g. by peylation, glycosylation, sulfatation, phosphorylation or any other modification, in particular of one or more surface or solvent exposed amino acid(s).

The neurotoxic component referred to herein, may be part of a composition. This composition may contain the neurotoxic component as the sole active component or may contain additional pharmaceutically active components.

Preferably, said composition comprises the neurotoxic component of Botulinum toxin type A. Said composition is a reconstituted solution of the neurotoxic component of Botulinum toxin. Preferably, the composition further comprises sucrose or human serum albumin or both, still more preferably the ratio of human serum albumin to sucrose is about 1:5. In one embodiment, the composition is Xeomin^{®}. More preferably, said human serum albumin is recombinant human serum albumin. Alternatively, said composition is free of mammalian derived proteins such as human serum albumin. Any such solution may provide sufficient neurotoxin stability by replacing serum albumin with other non-proteinaceous stabilizers (infra).

The composition may comprise additional components such as a pH buffer, excipient, cryoprotectant, preservatives, analgesics stabilizer or any combination thereof. The term "analgesic" relates to analgesic drugs that act in various ways on the peripheral and central nervous systems and includes inter alia paracetamol (acetaminophen), the nonsteroidal anti-inflammatory drugs (NSAIDs) such as the salicylates, narcotic drugs such as morphine, synthetic drugs with narcotic properties such as tramadol, and various others. Also included is any compound with a local analgesic effect such as e.g. lidocaine, benzylic alcohol, benzoic acid and others.

The term "lyophilization" is used in this document for a treatment of a solution containing the neurotoxic component of the Botulinum toxin, whereas this solution is frozen and dried until only the solid components of the composition are left over. The freeze-dried product of this treatment is therefore defined in this document as "lyophilisate".

In this document the term "reconstitution" is defined as the process of solubilization of said freeze-dried composition of the neurotoxic component. This can be done by adding the appropriate amount of sterile water, e.g. if all necessary components are already contained in the lyophilisate. Or, if this is not the case, it can be done e.g. by adding a sterile saline-solution alone or if applicable with the addition of components comprising e.g. a pH buffer, excipient, cryoprotectant, preservative, analgesic stabilizer or any combination thereof. The saline of before mentioned "saline-solution" is a salt-solution, more preferably being a sodium-chloride (NaCl) solution, still more preferably being an isotonic sodium-chloride solution (i.e. a sodium-chloride concentration of 0,9%). The solubilization is carried out in such a manner that the final "reconstitution" is directly or indirectly, i.e. for example after dilution, administrable to the patient. Preferably, the neurotoxin is reconstituted in isotonic media. More preferably in isotonicsaline. More preferably, said saline is sterile saline.

The terminology "freeze-and-thaw cycle" used in this document refers to a process of freezing and thawing of the reconstituted solution. Whereby the process of "freezing" is defined as the storage of the reconstituted solution at temperatures below 0°C, for example preferably below -20°C (normal freezer temperature), more preferably at a temperature of -80°C (dry ice temperature) or below. And whereby the process of "thawing" is defined as a storage above 0°C, preferably above +4°C, more preferably above +20°C, most preferably to the temperature ranges of above +25, +30, +40 respectively, but not above 50°C. Typical and exemplary storage times during and after freezing and thawing are up to 1 minute, up to 10 minutes, up to 30 minutes, up to 1 hour, up to 2 hours, up to 3 hours, up to 4 hours, up to 5 hours, up to 6 hours, up to 7 hours, up to 8 hours, up to 1 day, up to 2 days, up to 3 days, up to 4 days, up to 5 days, up to 6 days, up to 7 days, up to 8 days, up to 9 days, up to 10 days, up to 2 weeks, up to 3 weeks, up to 1 month, up to 2 months, up to 3 months (90 days). The terminology "freeze-and-thaw cycle" also includes the subsequent cooling down and (again) heating up cycle in a continuous manner. The time periods mentioned above are only typical examples and the actual time periods may be longer or shorter and include any interval between the numerical values given above. The definition of one "freeze-and-thaw cycle" is one freezing and one thawing step under the above mentioned conditions. The plural of "freeze-and-thaw-cycle", namely "freeze-and-thaw-cycles", as mentioned in this document refers to repetition of one "freeze-and-thaw cycle" with the reconstituted solution at both different as well as the same time-intervals and temperatures, as they are defined in the above ranges. The repetitions mentioned above can be for at least two times, more preferably at least three or at least four times, even more preferably at least five, at least six, at least seven times, even more preferably at least eight, at least nine or at least ten times, but not more than 20 times.

The term "pH buffer" refers to a chemical substance being capable to adjust the pH value of a composition, solution and the like to a certain value or to a certain pH range. In one embodiment this pH range can be between pH 5 to pH 8, more preferably pH 7 to pH 8, even more preferably 7,2 to 7,6, and most preferably a pH of 7,4. The pH ranges given mentioned above are only typical examples and the actual pH may include any interval between the numerical values given above. Suitable buffers which are in accordance with the teaching of the present invention are e.g. sodium-phosphate buffer, sodium-acetate buffer, TRIS buffer or any buffer, which is suitable to buffer within the above pH-ranges.

The terms "preservative" and "preservatives" refer to a substance or a group of substances, respectively, which prevent the growth or survival of microorganisms, insects, bacteria or other contaminating organisms within said composition. Preservatives also prevent said composition from undesired chemical changes. Preservatives which can be used in the scope of this patent are all preservatives of the state of the art known to the skilled person. Examples of preservatives that might be used include, inter alia, e.g. benzylic alcohol, benzoic acid, benzalkonium chloride, calcium propionate, sodium nitrate, sodium nitrite, sulphites (sulfur dioxide, sodium bisulfite, potassium hydrogen sulfite, etc.), disodium EDTA, formaldehyde, glutaraldehyde, diatomaceous earth, ethanol, methyl chloroisothiazolinone, butylated hydroxyanisole and/or butylated hydroxytoluene.The term "container" refers to a vessel, like a vial, a syringe, a flask or any other kind of reservoir in which said composition can be stored or transported or both. The walls of this vessel are in direct contact with the said composition and comprise of materials like all sorts of glass, plastic, metal, ceramic or any combination thereof, or any material, which is suitable to hold the reconstituted solution tightly.

"Stable", "stabilizing", "stabilizes" or "stabilization" means that the neurotoxic component in a reconstituted or aqueous solution of pharmaceutical composition has greater than about 20%, 30%, 40%, 50%, 60%, 70%, 80%, 90%, and up to about 100% of the toxicity that the biologically active neurotoxic component had prior to being incorporated into the pharmaceutical composition. "Cryoprotectant" refers to excipients which result in the neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being freeze-dried and reconstituted in the pharmaceutical composition.

Examples of such stabilizers are gelatin or albumin, preferably of human origin or obtained from a recombinant source. The stabilizers may be modified by chemical means or by recombinant genetics or both. In a preferred embodiment of the present invention, it is envisaged to use alcohols, e.g., inositol, mannitol, as cryoprotectant excipients to stabilize the neurotoxic component during lyophilization.

In another embodiment, the stabilizer may be a non-proteinaceous stabilizing agent comprising hyaluronic acid or polyvinylpyrrolidone or polyethylene glycol or a mixture of two or more thereof. Such composition is considered to be a composition possessing remarkable stability.

The term "room temperature" in this document refers to any temperature between +20°C to +25°C, even more preferably any of the temperatures of +20°C, +21°C, +22°C, +23°C, +24°C or +25°C and any value in between.

The term "excipient" in this document refers to a substance present in a pharmaceutical composition other than the active pharmaceutical ingredient present in the pharmaceutical composition. An excipient can be a buffer, carrier, antiadherent, binder, disintegrant, filler, diluent, preservative, vehicle, cyclodextrin and/or bulking agent, such as albumin, gelatin, collagen and/or sodium chloride.

The "device for cooling" is defined as any device being capable to reduce the temperature of the composition below the environmental temperature. Preferably said "cooling device" achieves a stable temperature below the environmental temperature, typically at or around 6°C, in some cases even below.

In a more preferred embodiment of the present invention, the composition may comprise the neurotoxic component and a hyaluronic acid or a polyvinylpyrrolidone or a polyethylene glycol or any combination thereof, such composition being optionally pH stabilized by a suitable pH buffer, in particular by a sodium acetate buffer, or a cryoprotectant polyalcohol or both.

Preferably, the neurotoxic component has a biological activity of 50 to 250 LD₅₀ units per ng neurotoxic component, as determined in a mouse LD₅₀ assay. More preferably, the neurotoxic component has a biological activity of about 150 LD₅₀ units per nanogram.

In another preferred embodiment, the composition may contain a polyhydroxy compound, e.g. a polyalcohol as cryoprotectant. Examples of polyalcohols that might be used include, e.g., inositol, mannitol and other non-reducing alcohols. Some embodiments of the composition do not comprise a proteinaceous stabilizer, preferably do not contain trehalose or maltotriose or lactose or sucrose or related sugar or carbohydrate compounds which are sometimes used as cryoprotectants.

The polyvinylpyrrolidone when present in the instant composition, is preferably combined with the instant neurotoxic component in such a quantity to provide a reconstituted solution comprising 10 to 500 mg, especially 100 mg polyvinylpyrrolidone per ml in a 200 U/ml neurotoxic component of botulinum toxin solution. In another preferred embodiment reconstitution is carried out in up to 8 ml solution. This results in concentrations of down to 12.5 mg polyvinylpyrrolidone per ml in a 25 U/ml neurotoxic component solution.

More preferably, the resulting solution also contains a 1-100 mM, especially 10 mM sodium acetate buffer.

The polyethylene glycol in the composition is preferably combined with the instant neurotoxic component in such a quantity to provide a reconstituted solution comprising 10 to 500 mg, especially 100 mg polyethyleneglycol per ml in a 200 U/ml neurotoxic component solution. More preferably, the resulting solution also contains a 1-100 mM, especially 10 mM sodium acetate buffer. This ratio of components is also applied in case lower concentrations of down to 25U/ml neurotoxic component solution.

Thus, in a preferred embodiment the neurotoxic component is formulated together with a hyaluronic acid stabilizer or a polyvinylpyrrolidone stabilizer or a polyethylene glycol stabilizer or any combination thereof. Additionally, the composition may contain a sodium acetate buffer system or an alcoholic cryoprotectant or both. In a further preferred embodiment the formulation is albumin free, and comprises as a stabilizer hyaluronic acid, polyvinylpyrrolidone (Kollidon®), hydroxyethyl starch or alginate or a mixture of two or more of these. Said preferred composition comprises in addition to the mentioned stabilizers water and at least one polyalcohol, preferably mannitol or sorbitol or mixtures thereof.

Typically, the above referenced provision of the muscle relaxant involves storage or transport or both of the same, or is a step within a process for preparing said muscle relaxant, respectively, at elevated temperatures, more preferably a step carried out after the proteins including the neurotoxic component of Botulinum toxin have been lyophylised and reconstituted. By "elevated temperatures" temperatures above +6°C, preferably above +20°C, more preferably above +30°C are meant. The term "above +6°C" means e.g. +7°C, +8°C, +9°C, +10°C, +11°C, +12°C, +13°C, +14°C, +15°C, +16°C, +17°C, +18°C, +19°C and or more, but not above 70°C. The term "above +20°C" means e.g. +21°C, +22°C, +23°C, +24°C, +25°C, +26°C, +27°C, +28°C, +29°C or +30°C. The term "above +30°C" means e.g. +31 °C, +32°C, +33°C, +34°C, +35°C, +36°C, +37°C, +38°C, +39°C or +40°C. Preferably the muscle relaxant is not stored above +70°C. In some cases, i.e. in an environment where the muscle relaxant on the basis of the neurotoxic component of Botulinum toxin is stored below 0°C, the term "elevated temperatures" refers to temperatures above 0°C, preferably above +4°C, and most preferably to the above recited temperature ranges of above +6°C, +20°C and +30°C, respectively.

In a preferred embodiment, the muscle relaxant is subjected to a temperature lying in the range of above +6°C and up to +40°C for a time period not exceeding 14 days. As the person skilled in the art is perfectly aware of, the time period for which the muscle relaxant is subjected to the respective temperature can be any time interval between a few minutes and 14 days. Typically, taking into account the circumstances of providing such a muscle relaxant, and in particular the situation where storing or transportation or both is involved the time period will not be less than 10 minutes. These short periods are particularly important under circumstances, wherein after transportation and before storage in a hot climate, the muscle relaxant is subjected to direct sunlight, e.g. on an airport or on the street. Typical time periods within the present invention are therefore, up to 10 minutes, up to 30 minutes, up to 1 hour, up to 3 hours, up to 4 hours, up to 5 hours, up to 6 hours, up to 7 hours, up to 8 hours, up to 1 days, up to 2 days, up to 3 days, up to 4 days, up to 5 days, up to 6 days, up to 7 days, up to 8 days, up to 9 days, up to 10 days, up to 2 weeks, up to 3 weeks, up to 1 month, up to 2 months, up to 3 months (90 days). Needless to say, that the time periods mentioned above are only typical examples and the actual time periods may be longer or shorter and include any interval between the numerical values given above.

As to the temperature, to which the muscle relaxant is subjected, typically a lower limit of a temperature of above 20°C is envisioned by the person skilled in the art. With respect to the temperatures and temperature ranges specified herein, the person skilled in the art understands that the upper temperature to which the muscle relaxant/composition is subjected is preferably not above 70°C. This is, the temperatures to which the muscle relaxant is subjected preferably lie in a range of above 20°C and up to 70°C. Therefore, within the present invention, the muscle relaxant is subjected to a temperature above 20°C, or above 25°C, or above 30°C, or above 35°C, or above 40°C, or above 45°C, or above 50°C, or above 60°C, or above 65°C, to up to 70°C, respectively. Again, any specific temperature between the given values of above 20°C and up to 70°C as well as respective temperature intervals, which may be the result of the environment at which the muscle relaxant is provided, preferably transported or stored or both, lies within the present invention.

The following temperature and time intervals represent preferred embodiments of the present invention. According to a first embodiment the muscle relaxant is subjected to a temperature above +30°C and up to +70°C for a time period not exceeding 90 days, more preferably to a temperature above +30°C and up to +70°C for a time period ranging from 10 minutes to 14 days, more preferably at a temperature of between +40°C and +60°C and a time period ranging from 10 minutes to 90 days.

In a further preferred embodiment the time period ranges from 10 minutes to 30 days, while the temperature ranges from above 30°C to up to 70°C, preferably from 40°C to 60°C, more preferably from 50°C to 60°C.

In a further preferred embodiment, representing extreme conditions, the temperature lies in the range of between 65°C and 70°C and the time period for which the muscle relaxant is subjected to said temperature lies in the range of from 10 minutes to 90 days, preferably from 10 minutes to 3 days.

The experimental results demonstrate that the surface to which the solution is exposed has no impact on the stability of the reconstituted neurotoxin of the present invention. Accordingly, the reconstituted composition of the present invention can be kept in various vessels or containers. The surfaces of such vessels or containers may thus be made of any kind of plastic, metal, glass etc.

Due to the findings on which the present invention is based, it is now possible to provide a muscle relaxant as outlined above without using a device for artificial cooling. This finding is particularly important for the transportation or storage or both of such a muscle relaxant. Furthermore, the invention is particularly relevant in an environment of elevated temperature, possibly together with an increased humidity.

The present invention is now further exemplified by way of the non-limited examples recited hereinunder.

### EXAMPLES

The examples have been conducted with the commercially available product Xeomin^{®}. Xeomin^{®} is a lyophilized powder containing Botulinum neurotoxin type A (150 kDa) as active ingredient. The toxin is present in nicked double chain form, i.e. it contains a heavy and a light chain. The toxin is obtained from *Clostridium botulinum* cultures (strain ATCC 3205). It has been purified to such a degree that it is free of any complexing proteins. Xeomin^{®} further comprises human serum albumin and sucrose.

For evaluating the stability of Xeomin^{®}, the biological activity was determined by using the mouse hemidiaphragm assay (HDA). In this assay, a nerve muscle preparation composed of the murine *nervus phrenicus* and the corresponding part of the diaphragm muscle is fastened in a force measurement apparatus. The intact phrenic nerve is threaded through two electrodes used to stimulate the nerve and thereby, the diaphragm. This composition is immersed in the toxin containing HDA buffer solution and the nerve is periodically stimulated by electric impulses (frequency 1 Hz, stimulus duration 0.1 ms, stimulation current amplitude 5-50 mA).

The contractile response of this indirectly stimulated muscle is detected with the aid of isometric transducers. The signal is amplified and documented using the commercially availabe software VitroDatWin 3.4 on a personal computer. When measuring the time course of the muscle contractile response, a exponential decrease of the contraction force is observed in the presence of botulinum toxin. A characteristic of this decrease is the so-called paralysis time. The paralysis time is defined as the time between the two time points "addition of the toxin sample" and "half maximum contraction force" and is proportional to the toxin concentration of the organ bath.

The above-mentioned methods were carried out in accordance with the requirements laid down in the European Pharmacopeia for testing of Botulinum toxin activity.

### EXAMPLE 1 "STORAGE AT ROOM TEMPERATURE"

Reconstituted saline solution of Xeomin^{®} was stored up to 9 days in plastic-syringes at room-temperature (+23°C). The activity of the samples was compared with references which had been reconstituted immediately (no longer than 2 hours) before activity determination. No significant reduction of protein-activity could be detected between the sample groups and the references.

### EXAMPLE 2 "STORAGE IN PLASTIC CONTAINERS"

Reconstituted Botulinum neurotoxin NT201 in saline solution was drawn into and applied from various plastic containers (cf. table 1 below). Additionally, reconstituted toxin was stored for different periods of up to 14 days in plastic containers and syringes, respectively, prior to activity measurement. In none of the cases a significant reduction of protein-activity could be detected.

**Table 1:**

| Plastic injection material | | |
|---|---|---|
| Pipette tips | Eppendorf AG | #0030 000.854 |
| | | #0030 000.870 |
| | | #0030 000.919 |
| | | #0030 000.978 |
| Microtube | Sarstedt AG | #72.690 |
| Centrifugal tube | Sarstedt AG | #62.554.001 PP |
| Cryovial | Nunc | #379146 |
| Syringe 1 ml | B. Braun | #9161406V |
| Syringe 1 ml | BD (Becton Dickinson) | #300013 (EU edition) |
| Syringe 1 ml | BD (Becton Dickinson) | #309602 (US edition) |
| Syringe 3 ml | BD (Becton Dickinson) | #300910 |
| Syringe 5 ml | B. Braun | #4617053V |
| Syringe 10 ml | BD (Becton Dickinson) | #300912 |
| Syringe adapter female / female | B. Braun | #5206634 |
| Needle 20G | BD (Becton Dickinson) | #301300 |
| Needle 21 G | B. Braun | #4565503 |
| Needle 21 G | BD (Becton Dickinson) | #301155 |
| Needle 28G | B. Braun | #4657683 |
| Needle 30G | B. Braun | #4656300 |
| Needle 30G | BD (Becton Dickinson) | #304000 |
| Syringe stopper | B. Braun | #4495101 |
| Syringe stopper | BD (Becton Dickinson) | #394075 |

The freeze-dried powder of NT201 containing 100 MLD ("median lethal dose" or LD₅₀ units) of toxin was reconstituted in saline solution prior to application.

To test the influence of plastic material on the activity of the botulinum neurotoxin, its reconstituted saline solution was drawn into and applied from the various syringes using the listed needles, adapters and stoppers listed in the table above and applied to the organ bath (the vessel in which the activity determination is performed by the mouse hemidiaphragm assay) using ordinary injection methods with the intention of simulating the medical procedure of intramuscular injection.

Additionally, reconstituted toxin was stored for different periods of up to 14 days in plastic containers in syringes, respectively, prior to activity measurement.

The experiments were performed in three independent series in order to study a maximum number of different variables and to get reliable results. Details are described in the following.

Saline aliquots were taken either directly with a pipette (series 1) or with 3 ml syringes and 20 G needles (BD #300910 and BD #301300; series 2) or with 5 ml syringes and 20 G needles (B. Braun #4617053V and #4665503; series 3) according to typical medical practice (series 2 and 3).

In order to ensure the accuracy of small sample volumes, the saline solution was injected into a centrifuge tube for intermediate storage when the required amount was less than 0.5 ml. In these cases, the desired volume was later transferred using a pipette.
- Series 1 & 3:: Ten vials were carefully aerated using a thin needle and subsequently opened. Saline (1.0 ml) was pipetted into the first vial. After fully dissolving the pellet by carefully mixing, the solution was transferred into the second vial. In this way, the fluid was transferred from one vial to the next until the last, i.e. the tenth vial. Then, any residual sample was collected by adding another 1.0 ml saline solution into the first vial and washing the vials one after another by the above described procedure and lastly combining both toxin solutions. These combined solutions (2.0 ml) represent the botulinum neurotoxin pool with about 100 MLD per 0.2 ml solution. When subsequently handled with syringes, 0.2 ml aliquots of the pool were pipetted into microtubes in order to guarantee accurate volumes.
- Series 2:: Exactly 2.5 ml saline solution were drawn into a 3 ml syringe with 20 G needle (BD #300910 and BD #301300) and injected in portions of 0.5 ml each into five vials containing the botulinum neurotoxin. The vials were carefully mixed until the whole pellet was dissolved. After aerating the vials by insertion of a thin needle, the reconstituted toxin was drawn into a 10 ml syringe with 21 G needle (BD #300912 and BD #301155). Air was then drawn into the syringe to a total volume of 10 ml and the syringe sealed with a stopper (#394075). Then the syringe was carefully rotated to allow the contact between the toxin solution and the whole syringe inner surface. The control sample pool was created as described above with the exception of using 0.5 ml saline solution per each of the five vials instead of 0.2 ml.
The experiments were performed in three blocks with three different botulinum toxin pools. Either 0.2 ml (series 1 and 3) or 0.5 ml (series 2) botulinum neurotoxin pool were used per sample corresponding, however, in each case to 100 MLD of botulinum toxin. This volume was either filled into microtubes and then drawn into a syringe or was directly stored in cryovials.
- Series 1:: In a first experiment, aliquots of the botulinum neurotoxin pool were either stored in cryovials or in 1 ml syringes with 26 G needles (BD #309602, US edition, and B. Braun #4657683) in the refrigerator for 7 and 14 days, respectively. The toxin activity measurement was started by injection and by pipetting the toxin aliquot (0.2 ml = 100 MLD) into the prepared hemidiaphragm organ bath containing 3.6 ml HDA buffer (Eagles balanced salt solution + 0.1 % human serum albumin). Residual toxin solution was rinsed from the syringes by drawing another 0.2 ml of fresh saline solution into the syringe and injected into the organ bath. The microtube was also rinsed with 0.2 ml of fresh saline solution using a pipette.

- Series 2:: The second series was performed using several different injection materials from Becton Dickinson. The control pool was either used immediately or stored for a maximum of 1 hour in a microtube on ice prior to activity determination. The above mentioned botulinum neurotoxin pool was stored at 4°C to 8°C inside a closed 10 ml syringe for 1 hour, 3 days and 5 days, respectively. Samples of 0.5 ml were taken by connecting a 1 ml syringe via a female/female adapter (BD #300013 and B. Brown #5206634) to the 10 ml storage syringe. After taking the sample the storage syringe was resealed and - as the case may be - returned to the refrigerator. The toxin activity measurement was started by injecting or pipetting the toxin aliquot (0.5 ml = 100 MLD) into the prepared hemidiaphragm organ bath containing 3.5 ml HDA buffer (Eagles balanced salt solution + 0.1 % human serum albumin). A 30 G needle (BD #304000) was used together with the above mentioned 1 ml syringe. Residual toxin was rinsed from the syringe by drawing 0.5 ml of the hemidiaphragm organ bath solution into the syringe and injecting it back into the organ bath.
- Series 3:: The final series was performed with injection material from B. Braun. The control pool was either used immediately or stored for a maximum of 1 hour in a microtube on ice prior to toxin activity determination. The above mentioned 0.2 ml botulinum neurotoxin pool aliquots were drawn into 1 ml syringes equipped with 20 G needles (B. Braun #9161406V and #4665503). Air was then drawn into the syringes to a total volume of 1 ml and the syringes sealed with stoppers (B. Braun #4495101). Then the syringes were carefully rotated to allow the contact between the toxin solution and the whole inner syringe surface. Afterwards, the samples were stored as 4°C to 8°C for 1 hour, 3 days and 9 days, respectively.

The toxin activity measurement was started by injection or pipetting the toxin aliquot (0.2 ml = 100 MLD) into the prepared hemidiaphragm organ bath containing 3.6 ml HDA buffer (Eagles balanced salt solution + 0.1 % human serum albumin). A 30 G needle (B. Braun #4656300) was used together with the above mentioned 1 ml syringe. Residual toxin ways rinsed from the syringe by drawing another 0.2 ml of fresh saline solution into the syringe and injecting it back into the organ bath.

The results of the activity determination are listed in the following table.

| | Sample | Paralysis | Mean | SD¹ |
|---|---|---|---|---|
| Series 1 | Control | 70 min | 70.0 min | - |
| | 7 days storage in cryovials | 73 min | 71.5 min | 2.1 min |
| | | 70 min | | |
| | 14 days storage in cryovials | 75 min | 76.0 min | 1.4 min |
| | | 77 min | | |
| | 7 days storage in syringes from BD (US edition) | 76 min | 71.3 min | 4.2 min |
| | | 68 min | | |
| | | 70 min | | |
| | 14 days storage in syringes from BD (US edition) | 70 min | 70.0 min | - |
| | | | | |
| | | | | |
| Series 2 | Control | 82 min | 74.3 min | 6.0 min |
| | | 70 min | | |
| | | 69 min | | |
| | | 76 min | | |
| | 1 hour storage in syringes from BD (EU edition) | 62 min | 66.0 min | 3.5 min |
| | | 68 min | | |
| | | 68 min | | |
| | 3 days storage in syringes from BD (EU edition) | 67 min | 63.5 min | 2.8 min |
| | | 60 min | | |
| | 5 days storage in syringes from BD (EU edition) | 64 min | 66.0 min | 4.9 min |
| | | 68 min | | |
| | | | | |
| Series 3 | Control | 68 min | 70.5 min | 3.5 min |
| | | 73 min | | |
| | 1 hour storage in syringes from B. Braun | 68 min | 65.5 min | 3.5 min |
| | | 63 min | | |
| | 1 day storage in syringes from B. Braun | 75 min | 75.0 min | - |
| | | | | |
| | 3 days storage in syringes from B. Braun | 64 min | 64.5 min | 0.7 min |
| | | 65 min | | |
| | 9 days storage in syringes from B. Braun | 74 min | 69.0 min | 7.1 min |
| | | 64 min | | |

| | | | | |
|---|---|---|---|---|
| (¹SD - standard deviation) | | | | |

The plastic materials used in this study show no significant effect on the activity of the reconstituted neurotoxin formulation.

The individual paralysis time values range from 68 min to 82 min (mean: 72.6 min ± 5.0 min) for control samples and from 60 min to 77 min (mean: 68.6 min ± 4.8 min) for samples treated with plastic material.

### EXAMPLE 3 "STABILITY IN PRESENCE OF PRESERVATIVE"

Xeomin® was subjected to reconstitution in sterile saline solution with or without preservative (benzylic alcohol) and stored for various time ranges. The pools were stored in polyethylene vessels at 4°C for up to 14 days. No significant reduction of protein-activity could be detected in the presence of benzylic alcohol.

Each vial of drug product was reconstituted with 1.0 ml saline (with (0.9 % v/v) or without preservative) to a final concentration of 100 MLD/ml and stored in polyethylene vessels for the appropriate storage time. All samples were then pooled according to test group.

Due to the narcotic action of benzylic alcohol on the hemidiaphragm preparation of the testing system, a dialysis step was required to remove this agent prior to this bioassay. Therefore, irrespective of the presence or absence of this preservative, all test samples were dialyzed twice against a 250fold excess of Earl's buffered salt solution (EBSS) at 4°C for more than 2 hours each.

Subsequent to the dialysis step, samples were diluted to the final concentration of 25 MLD/ml with EBSS, the residual activity of these samples was determined in the hemidiaphragm assay with a nominal dose of 100 MLD per measurement.

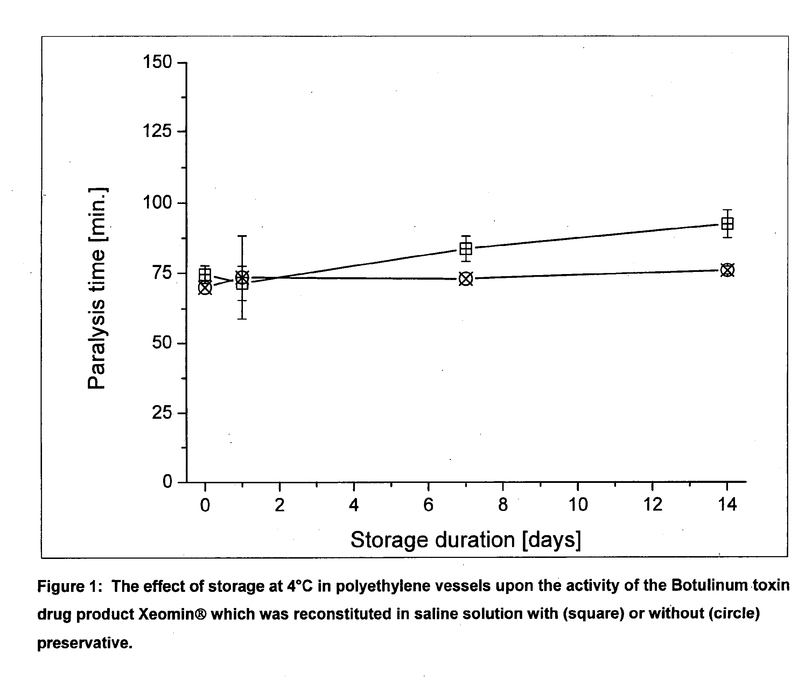

The results of this example are displayed in Figure 1.

Figure 1 shows the effect of storage at 4°C in polyethylene vessels upon the activity of the Botulinumtoxin drug product Xeomin^{®} which was reconstituted in saline solution with or without preservative.

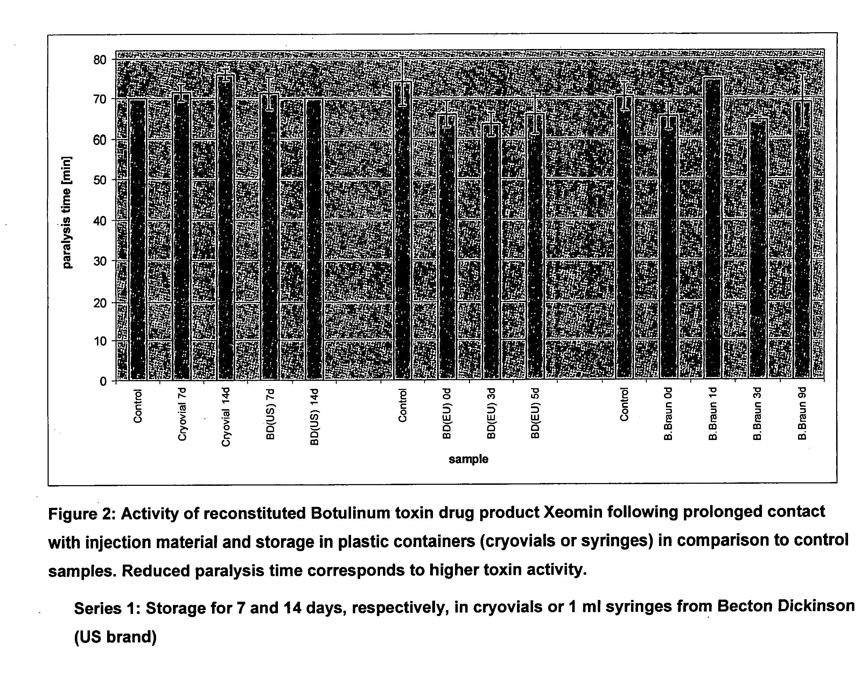

Figure 2 shows the effect. of storage at 4°C in polyethylene syringes with rubber stoppers upon the activity of the Botulinum drug product Xeomin® which was reconstituted in saline with or without preservative.

The results displayed in Figure 1 show that the reconstitution of Xeomin^{®} in saline solution with or without the preservative benzylic alcohol has no significant effect of the activity of the Botulinum Neurotoxin drug product.

The drug product is stable for up to 14 days when reconstituted in saline solution and stored at 4°C. The presence or absence of the preservative, benzylic alcohol, has no effect on this stability.

### EXAMPLE 4 "FREEZE AND THAW CYCLES"

In this example, Xeomin® was reconstituted in sterile saline solution without preservatives and frozen and thawed repeatedly up to five times. No significant effect on the paralytic activity of the botulinum neurotoxin drug product could be detected.

Sterile saline solution consists of 0.9% sodium chloride (w/vol) in water for injection purposes. The drug product Xeomin^{®} was employed to create a sample pool.

A total of fourteen vials of Xeomin^{®} in two groups of seven vials were carefully aerated using a needle and subsequently opened. Care was taken that the freeze-dried product was fully intact at the bottom of the vial.

The first vial of a seven vial Xeomin^{®} group was reconstituted with 1.4 ml saline solution and the freeze-dried product dissolved completely. This solution was transferred quantitatively into the next vial where again the lyophilisate was completely dissolved. This procedure was repeated until the drug product of all seven vials of the group was dissolved in 1.4 ml of sterile saline. These 1.4 mf of both groups were then pooled to create 2.8 ml Xeomin^{®}-pool.

Aliquots (0.2 ml) of this Xeomin^{®}-pool were pipetted into twelve labeled polypropylene tubes: two reference and 10 freeze-thaw duplicate samples numbered 1 to 5 (indicating the number of freeze-thaw-cycles). The two reference tubes were stored at 4°C until measured in the hemidiaphragm assay whereas the other tubes were frozen at -20°C for at least 120 minutes. The tubes labeled with 2-5 were completely thawed at room temperature (approx. 30 minutes) and subsequently frozen at -20°C for 120 minutes. This procedure was continued until the tubes labeled with 3 had been frozen for three times, the tubes labeled with four for four times and the tubes labeled with five for five times.

Prior to the hemidiaphragm assay, all samples were thawed at room temperature for thirty minutes and incubated at 37°C for 10 minutes. They were mixed and centrifuged for 10 seconds. Each sample was transferred to 3.6 ml Earl's buffered salt solution / 0.1% HSA using an additional 0.2 ml Earl's buffered salt solution / 0.1 % HSA to complete the transfer. The paralytic activity of the resulting 4.0 ml of Xeomin^{®} were subsequently measured in the hemidiaphragm assay.

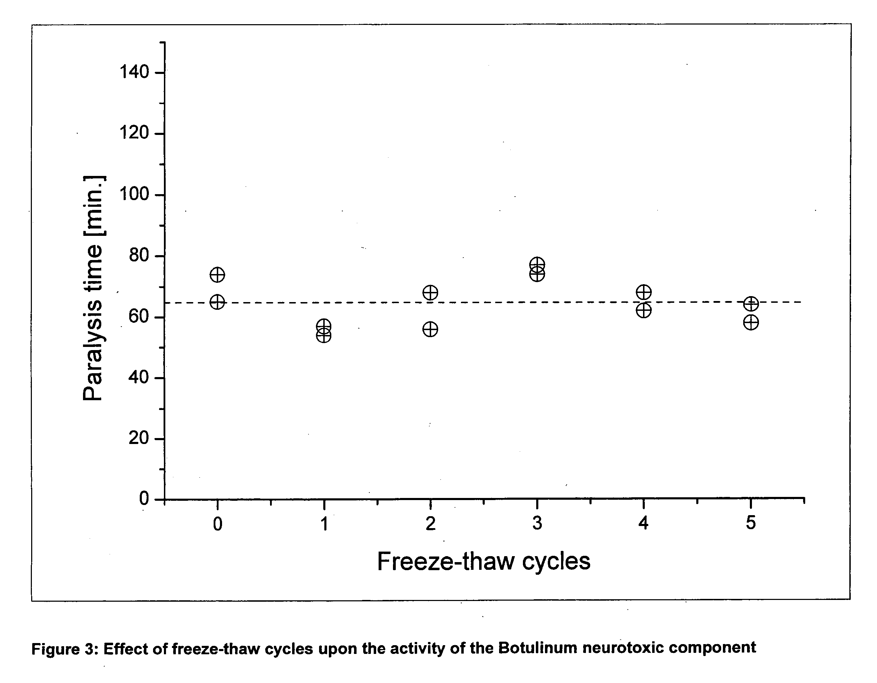

The results of these examples are displayed in Figure 3.

The results displayed in Figure 3 show that repeated freezing and thawing of reconstituted Xeomin^{®} has no significant effect on the paralytic activity of the botulinum neurotoxin drug product. Xeomin^{®} is stable for at least up to five freeze and thaw cycles when reconstituted in saline solution.

## Claims

1. Process for providing a muscle relaxant at temperatures above 6°C, preferably above 25°C, wherein said muscle relaxant is a reconstituted solution comprising the neurotoxic component of Botulinum toxin free of complexing proteins.

2. Process according to claim 1, wherein said provision involves storage and/or transport or is a step within a process for preparing said muscle relaxant.

3. Process according to claim 1 or 2, wherein the muscle relaxant is transported or stored or both without any device for cooling at an environmental temperature above 6°C, preferably above 25°C to up to 70°C.

4. Process according to any of the preceding claims, wherein the muscle relaxant is subjected to "freeze and thaw" - cycles.

5. Process according to claim 4, wherein the number of said "freeze and thaw" - cycles is from 1 to 20.

6. Process according to any of the preceding claims, wherein the muscle relaxant is stable in the presence of a preservative and/or analgesic.

7. Process according to any of the preceding claims, wherein said reconstituted solution is stored in containers made of plastic, glass or metal or any combination thereof.

8. Process according to any of the preceding claims, wherein the solution further comprises sucrose or human serum albumin or both.

9. Process according to any of the preceding claims, wherein the solution further comprises at least one component selected from the group consisting of a cryoprotectant, a stabilizer, a pH buffer, an excipient, different from sucrose and human serum albumin, respectively, and mixtures thereof.

10. Process according to any preceding claims, wherein the neurotoxic component is the neurotoxic component of Botulinum toxin type A.
